# EUROPEAN PATENT APPLICATION

(11) **EP 2 724 682 A1**
(43) Date of publication of application: **30.04.2014**
(21) Application number: 13189891.8
(22) Date of filing: 23.10.2013
(51) Int. Cl.: A61B 17/88

(54) **Fastener retention system**

(30) Priority: 23.10.2012 US 201261717265 P
(71) Applicant: Nexus Spinal, LLC, Salt Lake City, UT 84121 (US)
(72) Inventor: Hawkes, David T., Salt Lake City, Utah 84121 (US); Crocker, Kevin, Salt Lake City, Utah 84121 (US)
(74) Representative: Adam, Holger

(57) **Abstract**

A fastening system (10) comprises a driving tool (12) having a driving shaft and a fastener (16) having a fastener shaft. A sleeve (14) is sized and shaped to create an interference fit when installed about the driving shaft of the driving tool. The sleeve is also sized and shaped to create an interference fit when installed about the fastener shaft of the fastener.

## Description

### PRIORITY CLAIM

Priority is claimed to co-pending U.S. Provisional Patent Application Serial No. 61/717,265, filed October 23, 2012, which is hereby incorporated by reference in its entirety.

### BACKGROUND

### Field of the Invention

The present invention relates generally to the field of fasteners and fastener retainers.

### Related Art

For practically every application of a driver to a screw, a self-retaining mate between the two components would be advantageous. This is especially true in orthopedic surgery, where the screws are driven into bone through small surgical access windows or ports. To this end, numerous and various attempts have been made to provide a reliable and robust retention feature for driving fasteners into strong surfaces. However, such prior attempts have proved ineffective in one design consideration or another.

### SUMMARY OF THE INVENTION

In accordance with one aspect of the invention, a fastener retaining device is provided, including a sleeve, at least partially formed from an elastic material. The sleeve can have i) a first end sized and shaped to create an interference fit about a portion of an driver tool, and a ii) second end sized and shaped to create an interference fit about a driving portion of a fastener. Fitting of the sleeve about the driver tool and fitting of the sleeve about the fastener can secure the fastener to the driver tool prior to and during installation of the fastener.

In accordance with another aspect of the invention, a fastening system is provided, including a driving tool having a driving shaft and a fastener having a fastener shaft. A sleeve can be sized and shaped to create an interference fit when installed about the driving shaft of the driving tool, and can be sized and shaped to create an interference fit when installed about the fastener shaft of the fastener.

In accordance with another aspect of the invention, a method of installing a fastener in a base material is provided, including: installing a sleeve about a portion of a driver tool so as to create an interference fit between the driver tool and the sleeve; installing the sleeve about a portion of a fastener so as to create an interference fit between the sleeve and the fastener wherein the fastener and the driving tool mate within a central bore of the sleeve; driving the fastener into the base material with the driver tool; and removing the sleeve from the portion of the fastener after the fastener is driven into the base material.

Additional features and advantages of the invention will be apparent from the detailed description which follows, taken in conjunction with the accompanying drawings, which together illustrate, by way of example, features of the invention.

### BRIEF DESCRIPTION OF THE DRAWINGS

The following drawings illustrate exemplary embodiments for carrying out the invention. Like reference numerals refer to like parts in different views or embodiments of the present invention in the drawings.
**FIG. 1A** is a side, exploded view of a fastener driving system in accordance with an embodiment of the invention;
**FIG. 1B** is a side, assembled view of the fastener driving system of FIG. 1A;
**FIG. 1C** is a sectional view of the assembled system of FIG. 1B, taken along section C-C of FIG. 1B;
**FIG. 2A** is a side view of an exemplary retaining sleeve in accordance with an embodiment of the invention;
**FIG. 2B** is an end view of the sleeve of FIG. 2A;
**FIG. 2C** is a sectional view of the sleeve of FIG. 2A, taken along section C-C of FIG. 2A; and
**FIG. 3** is a flow chart illustrating an exemplary method of employing the present technology.

### DETAILED DESCRIPTION

Reference will now be made to the exemplary embodiments illustrated in the drawings, and specific language will be used herein to describe the same. It will nevertheless be understood that no limitation of the scope of the invention is thereby intended. Alterations and further modifications of the inventive features illustrated herein, and additional applications of the principles of the inventions as illustrated herein, which would occur to one skilled in the relevant art and having possession of this disclosure, are to be considered within the scope of the invention.

### Definitions

As used herein, the singular forms "a" and "the" can include plural referents unless the context clearly dictates otherwise. Thus, for example, reference to "a fastener" can include one or more of such fasteners.

As used herein, the terms "attached," "coupled," fixed," etc., can be used to describe a condition in which two or more components are coupled to one another in such a manner that they function as intended: that is, the force required to uncouple the components is sufficiently large such that the components will remain attached to one another during the service for which they were designed. Unless indicated to the contrary, such "coupled" components can be separable if sufficient force is applied to the components. In some aspects of the invention, components are elastically fixed or coupled to one another and will remain fixed during the useful life of the product for which they are designed; however, they may be uncoupled from one another using an appropriate level of force (applied in an appropriate manner and location), and will return to an original configuration (e.g., a condition, state, shape, size, etc.), which existed prior to the components being coupled to one another.

As used herein, the term "interference fit" shall be interpreted broadly as including the joining of any two mating parts such that one or the other (or both) parts slightly deviate in size from their nominal dimension, thereby deforming such part slightly, each being compressed, the interface between two parts creating a union of extremely high friction. The word "interference" refers to the fact that one part slightly interferes with the space that the other is occupying in its nominal dimension. As used herein, an interference fit is typically distinct from a compression fit, a threaded fit, etc.

As used herein, the term "substantially" refers to the complete or nearly complete extent or degree of an action, characteristic, property, state, structure, item, or result. As an arbitrary example, an object that is "substantially" enclosed would mean that the object is either completely enclosed or nearly completely enclosed. The exact allowable degree of deviation from absolute completeness may in some cases depend on the specific context. However, generally speaking the nearness of completion will be so as to have the same overall result as if absolute and total completion were obtained. The use of "substantially" is equally applicable when used in a negative connotation to refer to the complete or near complete lack of an action, characteristic, property, state, structure, item, or result. As another arbitrary example, a composition that is "substantially free of" an ingredient or element may still actually contain such item as long as there is no measurable effect thereof.

As used herein, the term "about" is used to provide flexibility to a numerical range endpoint by providing that a given value may be "a little above" or "a little below" the endpoint.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary.

Numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "about 1 to about 5" should be interpreted to include not only the explicitly recited values of about 1 to about 5, but also include individual values and sub-ranges within the indicated range. Thus, included in this numerical range are individual values such as 2, 3, and 4 and sub-ranges such as from 1-3, from 2-4, and from 3-5, etc., as well as 1, 2, 3, 4, and 5, individually.

This same principle applies to ranges reciting only one numerical value as a minimum or a maximum. Furthermore, such an interpretation should apply regardless of the breadth of the range or the characteristics being described.

As used herein, the term "superelastic" or "superelasticity" (also known as "pseudoelasticity"), is to be understood to apply to a material capable of an elastic (reversible) response to an applied stress, caused by a phase transformation between the austenitic and martensitic phases of a crystal. This phenomenon is exhibited in many shape-memory alloys. Pseudoelasticity results from the reversible motion of domain boundaries during the phase transformation, rather than just bond stretching or the introduction of defects in the crystal lattice (thus it is not "true" superelasticity but rather pseudoelasticity). Even if the domain boundaries do become pinned, they may be reversed through heating.

Thus, a pseudoelastic material may return to its previous shape (hence, shape memory) after the removal of even relatively high applied strains. One special case of pseudoelasticity is called the Bain Correspondence. This involves the austenite/martensite phase transformation between a face-centered crystal lattice and a body-centered tetragonal crystal structure.

Superelastic alloys belong to the larger family of shape-memory alloys. When mechanically loaded, a superelastic alloy deforms reversibly to very high strains - up to 10% - by the creation of a stress-induced phase. When the load is removed, the new phase becomes unstable and the material regains its original shape. Unlike shape-memory alloys, no change in temperature is needed for the alloy to recover its initial shape.

### Invention

The present invention relates generally to systems for retaining fasteners on a driver tool prior to and while the fastener is driven into a base material by the driver tool.

For nearly every application wherein a driver tool is utilized to install a threaded fastener (e.g., screw, lag-bolt, etc.), a self-retaining mate between the two components can be advantageous. This is particularly true in orthopedic surgery, where the fasteners/screws are driven into bone through small surgical access windows which may be referred to hereinafter as surgical ports. Further, particularly with regard to driving the fasteners/screws into bone or other hard substrates, high forces may be exerted to ensure proper gripping of the threads into the bone during the driving process as well as ensuring that the driving tool is properly mated with the driver head of the threaded fastener. Therefore, due to the high forces involved, great injury may be inflicted upon the patient should the driving tool slip from the fastener head and strike the patient. To this end, various attempts have been made to provide a reliable and robust retention feature for driving fasteners.

The present invention overcomes the shortcomings of the prior art. Namely, the present invention provides stability in various degrees of motion, withstands repeated use without degradation in performance, is simple to employ, and is inexpensive to manufacture.

Some prior art retention mechanisms allow the interface to wobble. This is usually the result of employing line contact interference between the driver tip and the screw head. The line contact provides retention, but not stability.

Some retention mechanisms fail under torque application. This is often the product of employing spring mechanisms for retention, which cannot withstand large stress concentration because the springs often have extremely thin cross sections.

Some mechanisms provide only a limited amount of retention force, which can easily fail during the critical moment of screw contact with the substrate or base material.

While some prior art technologies have attempted to use an external, actuated sleeve, many such prior sleeves have proven excessively bulky for use in orthoscopic application. The prior art sleeves have used various locking mechanisms which have proven cumbersome to actuate, and expensive to manufacture.

The present invention provides a fastener retaining system that retains fasteners on a driver prior to and during installation of the fastener. The present technology is advantageously small, robust, reliable, inexpensive, and simple to deploy.

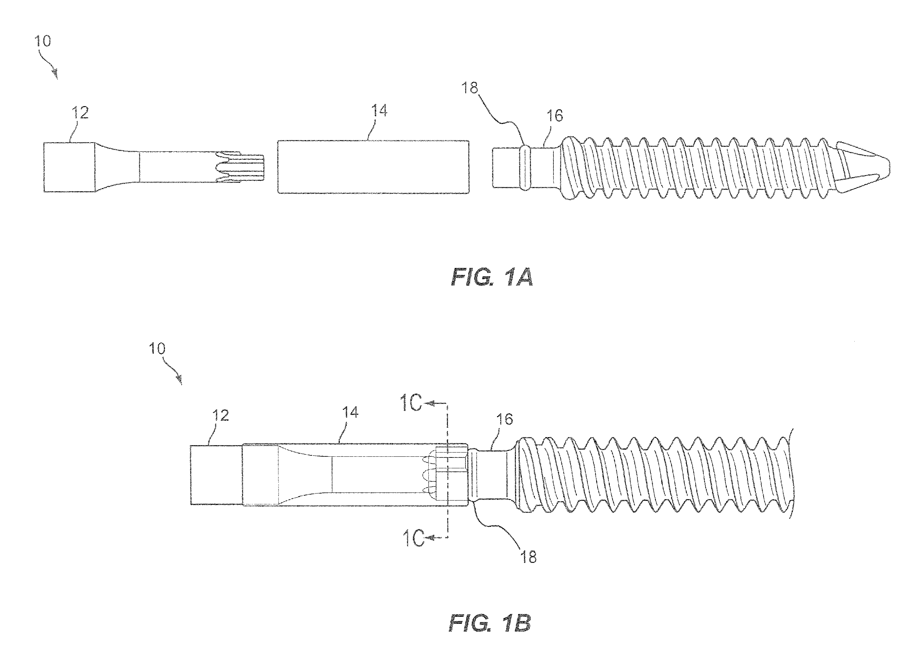

As illustrated in FIGs. 1A and 1B, a fastener and retainer system 10 in accordance with the present invention is provided that can include a driver or driver tool 12 (e.g., a torx bit driver, screw driver, nut driver, or the like). A fastener 16 is also provided that may take any number of forms, including, without limitation, a pedicle screw for use in surgical applications, which medical procedures may include procedures involving spinal surgery wherein pedicle screws are driven into the spinal column of a patient. The fastener may also include common construction applications such as drywall screws or concrete anchors, as well as common manufacturing applications wherein threaded fasteners may be driven into hard or dense substrates.

In the example shown in FIGs 1A and 1B, the fastener 16 is shown as a pedicle screw for use in spinal surgical applications. A retaining sleeve 14 is provided that is configured to provide a secure connection between the fastener 16 and the driver 12. FIG. 1B shows the fastener 16 being secured to the driver 12 within the sleeve 14 (note that the sleeve is shown as semi-transparent for clarity of viewing - the sleeve may or may not be transparent). The sleeve 14 retains the fastener 16 on the driver tip prior to and during installation of the fastener 16 into a base material or substrate (not shown).

Generally, an interference fit is provided between the sleeve 14 and a portion of the driver 12, as well as between the sleeve 14 and a portion of the fastener 16. While not so required, in one embodiment of the invention, the interference fit provided between the portion of the driver 12 and the sleeve 14 requires a greater magnitude of force to uncouple than the force required to uncouple the interference fit between the fastener 16 and the sleeve 14. In other words, the interference fit at the first end of the sleeve between the sleeve 14 and the driver 12 is intended to be stronger than the interference fit at the second end of the sleeve between the sleeve 14 and the fastener 16. In this manner, after the fastener 16 has been driven into the substrate and the tool 12 is pulled away, the sleeve 14 is retained on the tool 12 rather than being retained on the head of the fastener 16 as the driver tool 12 pulling free from it. Thus, the sleeve 14 can be substantially semi-permanently attached to the driver 12 and can be repeatedly mated with one or more fasteners 14.

Typically, as long as the interference fit between the driver 12 and the sleeve 14 is stronger than that between the sleeve 14 and the fastener 16, the driver 12 and sleeve 14 can easily be withdrawn from the fastener 16 while the fastener 16 remains installed within (or partially installed within) the base material or substrate. This provides the added advantage of greatly reducing the likelihood of the sleeve 14 being accidentally left behind in a patient upon completion of the surgical procedure. Additionally, if the sleeve 14 is retained upon the driver tool 12 it further makes the driver tool 12 instantaneously ready to couple to a subsequent new fastener in order to drive subsequent fasteners with ease resulting in little or no down time between applications.

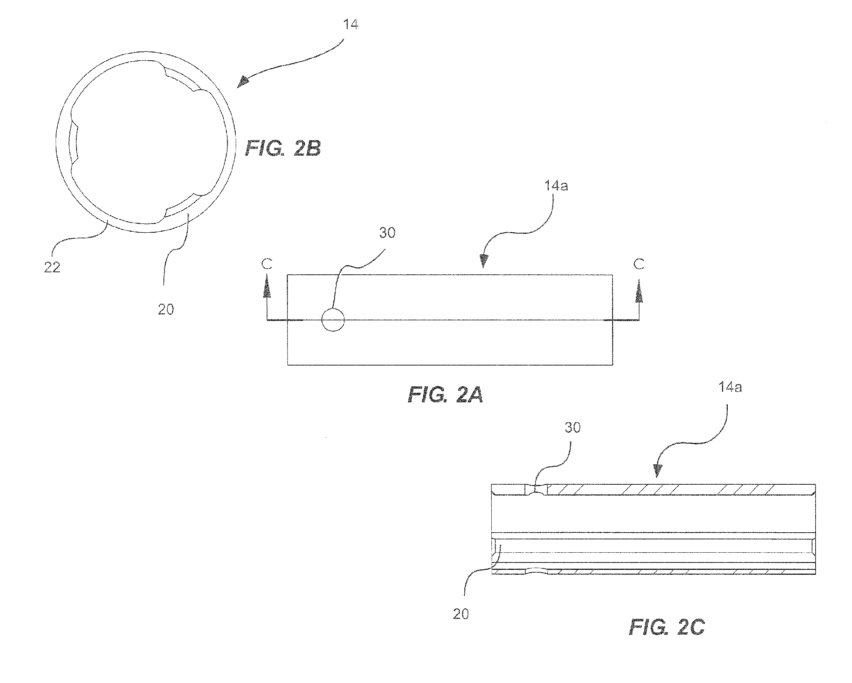

In another embodiment, the sleeve 14 can be either permanently or substantially permanently coupled to the driver 12 in manners other than an interference fit. As the sleeve 14 may not need to be removed from the driver 12, it can be permanently welded, bonded, pinned or otherwise formed as a part of the driver 12. The sleeve 14 can also be temporarily coupled to the driver 12 through manners other than an interference fit: such as by applying a pin through the holes 30 shown in FIGs. 2A and 2D.

As further shown in FIGs 1A-1B, the fastener may further be provided with a lip 18 indicating the proper depth to which the fastener should encase the outer perimeter of the head of the fastener. The lip may provide a visual indicator regarding whether the driver 12 is properly mated into the head of the fastener 16, wherein a gap between the lip and the sleeve indicates that the driver 12 has not properly engaged the corresponding slot in the fastener 16.

In one aspect of the invention, the sleeve 14 can be formed from an elastic material. Forming the sleeve 14 from an elastic material may allow the sleeve 14 to expand or otherwise deform about the mating portion of the driver 12 as well as about the mating portion of the fastener 16. In one aspect of the invention, the sleeve can be formed from a superelastic material. By employing a superelastic material, the sleeve provides an interface to withstand large tolerance and force variations without experiencing plastic deformation.

The use of superelastic materials allows for the sleeve 14 to be repeatedly deformed within the elastic range to provide the necessary interference fit for numerous fasteners without the need for replacing the sleeve 14 after every use. Additionally, the superelasticity of the material allows the sleeve 14 to elastically deform during each use without wearing out as quickly as would a sleeve formed out of a material that would need to plastically deform to provide the same measure of strength of the interference bond or fit.

The superelastic sleeves discussed herein take advantage of their large, reversible deformation. Examples of superelastic materials suitable for use in the present invention include, without limitation, nickel titanium otherwise known as nitinol. Other superelastic materials may also be suitable, including various other known superelastic polymers, alloys, etc.

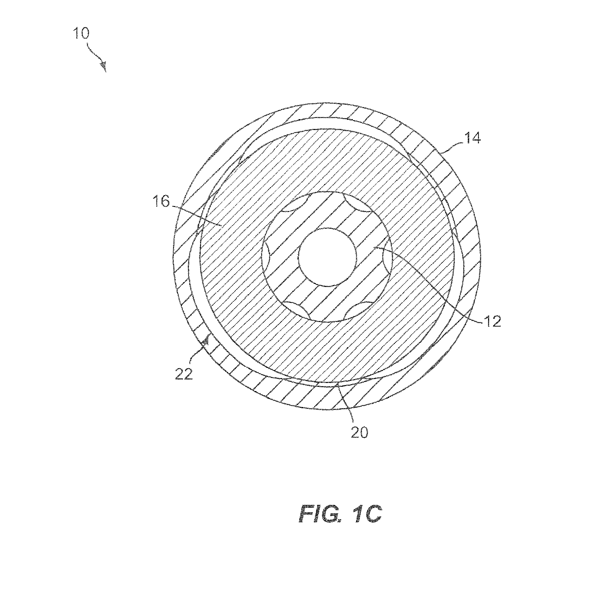

As seen in FIGs. 1C and 2A through 2C, in one aspect of the invention, the sleeve 14 is provided as an annular cylinder. The annular cylinder may have a central bore having an interrupted geometry and/or varying wall thickness provided. The varying wall thickness acts to provide a measure of control regarding the amount of force needed for assembly of the sleeve 14 to the driver 12 and the amount of retention provided by the sleeve 14 to the fastener 16. The cross section of FIG. 1C shows the internal driver 12, the fastener 16, with the external sleeve 14 cooperating with the driving tool 12 to provide a self-retaining coupling between fastener/screw 16 and the driver 12 during the driving process.

As also shown in these figures, varying the wall thickness of the sleeve 14, 14a can be accomplished by forming alternating thinner wall sections 20 in the wall (conversely, alternating thicker wall sections 20 can also be formed). In the example shown in FIG. 1C, the wall of the sleeve can include thin sections 20, which may be referred to herein as notches. The notches may be evenly spaced about the inner wall of the sleeve 14. These notches may also define thicker sections 20 of the wall, which may also be referred to herein as lobes or protrusions which protrude radially inward towards the open center of the annular sleeve 14.

The lobes 20 can provide contact with the portion of the fastener 16 (and/or the portion of the driver), while the thin sections 22 provide greater flexibility to the wall than do the lobe sections 20. For example, the effective inner diameter of the lobes 20 may be smaller than the outer diameter of the fastener head and the driver. As the sleeve is forced over these larger diameters, the thin sections 20 may elongate, i.e. stretch, to allow for the lobes to extend past the diameter of the fastener head and driver tip. Alternatively, the thin sections 20 may be straightened and slightly deform out of their semi-circular shape, wherein the sleeve 14 may more closely resemble a hexagon in its elastically deformed shape, in order to allow for the lobes 22 to extend about the outer diameters of the fastener head and driver tip.

Additionally, the lobes 20 may be tapered from one end to the other to have a larger effective diameter at the second fastener end and smaller effective diameter at the first or driver end. In this manner, more deformation is required to extend the sleeve 14 over the diameter of the driver 12 and less deformation to extend the sleeve 14 over the diameter of the fastener 16. Thus, a stronger interference fit can be created between the driver 12 and the sleeve 14 than between the fastener 16 and the sleeve 14. Additionally, an increasingly strong interference fit may be provided to the fastener 16 as the fastener 16 is pushed farther into the sleeve 14.

Another advantage realized by having the lobes 20 and the thinner sections 22 is that they allow an interference fit to be achieved and a secure coupling between the driver tool 12 and the fastener 16, even when the driver tool 12 cannot be properly positioned to be perfectly co-axial with the fastener 16. The interference fit between the sleeve 14 and the fastener 16 allows for a reduced force to be applied by the operator during the driving process and thus results in less wobble and less out-of axis deformation of the pedicle screw as it is driven into the bone.

In the example shown in FIGs. 1A-C, the fastener 16 is shown as a pedicle screw for use in surgical applications. It should also be appreciated that the fastener 16 may also be an anchor to attach other hardware to an already installed pedicle screw. It should be further appreciated that the fastener 16 may be any type of fastener provided by a certain industry and similar sleeves may be appreciated to work with any number of fasteners where it may be desired that the fastener be securely retained onto the head of a driving tool.

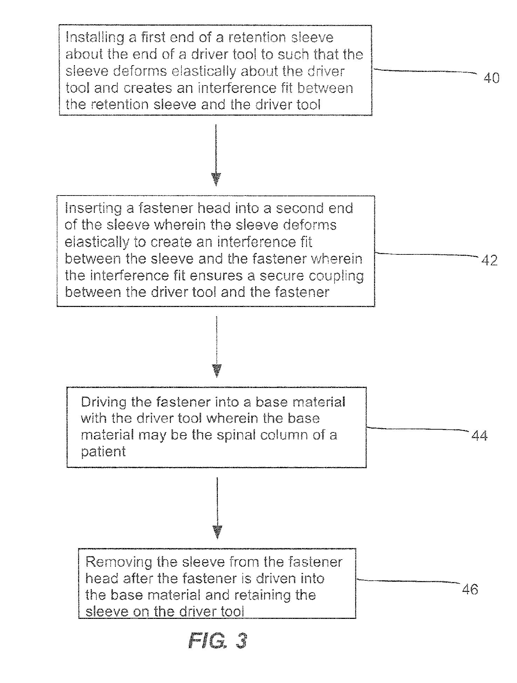

FIG. 3 depicts a method of installing a fastener using the fastener retention system of the present invention. The method of installing a fastener may include a first step 40 which includes installing a first end of a sleeve about an end portion of a driver tool so as to create an interference fit between the driver tool and the first end of the sleeve. The method may also include a second step 42 which involves inserting a fastener head into a second end of the sleeve wherein the sleeve deforms elastically around the head of the fastener so as to create an interference fit between the second end of the sleeve and the head of the fastener. This second interference fit can be configured to guide the fastener head a certain distance into the sleeve in order to mate with the driver tool and provide a secure mate between the driver tool and the head of the fastener head.

A third step 44 involves driving the fastener into a base material with the driver tool. This base material may be any suitable material. However, for purposes of the present embodiment, the base material can be the spinal column of a patient, or another part of a patient's anatomy. A fourth step 46 may involve removing the sleeve from the portion of the fastener after the fastener is driven into the base material. The removal step may include retaining the sleeve on the driving tool so as to ensure it is not left behind or otherwise dropped inside the patient, particularly in orthoscopic applications.

It is to be understood that the above-referenced arrangements are illustrative of the application for the principles of the present invention. Numerous modifications and alternative arrangements can be devised without departing from the spirit and scope of the present invention while the present invention has been shown in the drawings and described above in connection with the exemplary embodiments(s) of the invention. It will be apparent to those of ordinary skill in the art that numerous modifications can be made without departing from the principles and concepts of the invention as set forth in the examples.

## Claims

1. A fastener retaining device, comprising:
a sleeve, at least partially formed from an elastic material;
the sleeve having:
a first end sized and shaped to create an interference fit about a portion of a driver tool, and
a second end sized and shaped to create an interference fit about a portion of a fastener;
wherein fitting of the sleeve about the driver tool and fitting of the sleeve about the fastener secures the fastener to the driver tool prior to and during installation of the fastener.

2. The device of claim 1, wherein the interference fit between the first end of the sleeve with the driver tool requires a higher magnitude of force to uncouple than a force required to uncouple the interference fit between the second end of the sleeve and the fastener.

3. The device of claim 1, wherein the sleeve is at least partially formed from a superelastic material.

4. The device of claim 3, wherein the sleeve is formed from nitinol.

5. The device of claim 1, wherein the sleeve includes a plurality of lobes extending from an interior wall of the sleeve radially inward toward the center of the sleeve, the lobes defining notches therebetween and being spaced about a circumference of the sleeve.

6. The device of claim 1, wherein the sleeve includes an annular cylinder having a plurality of notches formed on an inner surface thereof, the plurality of notches being spaced about a circumference of the sleeve to thereby define a plurality of protrusions therebetween, the protrusions being operable to contact a portion of the fastener.

7. A fastening system, comprising:
a driving tool having a driving shaft;
a fastener having a fastener shaft; and
a sleeve, having a first end and a second end, the first end being sized and shaped to create an interference fit when coupled to the driving shaft of the driving tool, the second end being sized and shaped to create an interference fit when coupled to the fastener shaft of the fastener.

8. A method of installing a fastener in a base material, comprising:
installing a first end of a sleeve about an end portion of a driver tool so as to create an interference fit between the driver tool and the first end of the sleeve;
inserting a fastener head into a second end of the sleeve so as to create an interference fit between the second end of the sleeve and the head of the fastener, the sleeve being thereby configured to provide a secure coupling between the driver tool and the head of the fastener;
driving the fastener into a base material with the driver tool; and
removing the sleeve from the fastener head after the fastener is driven into the base material.

9. The method of claim 8, wherein removing the sleeve from the portion of the fastener further includes retaining the sleeve's interference fit about the end portion of the driver tool while removing the sleeve from the head of the fastener.
